# EUROPEAN PATENT SPECIFICATION

(11) **EP 0 440 427 B1**
(45) Date of publication and mention of the grant of the patent: **08.06.1994**
(21) Application number: 91300682.1
(22) Date of filing: 30.01.1991
(51) Int. Cl.: A61M 25/00

(54) **A catheter packaging system**
Verpackungssystem für Katheter
Système d'emballage pour cathéter

(30) Priority: 30.01.1990 IE 33190
(43) Date of publication of application: 07.08.1991
(73) Proprietor: BARD CONNAUGHT, Dublin 1 (IE)
(72) Inventor: Smyth, Frieda Maria, Salthill, Galway (IE)
(74) Representative: McCarthy, Denis Alexis

(56) References cited:
- US-A- 4 779 727

## Description

The present invention relates to packaging for catheters.

Catheters are generally provided as presterilised, prepackaged units in which the catheter is sealed in a package containing a tray for retaining the catheter shaft and an insert for retaining the catheter curve. Removal of the catheter from such a package frequently causes damage both to the curve and shaft regions of the catheter. This is particularly so in the case of long catheters (e.g. 100-130 cm).

Two methods of withdrawing a catheter from a sealed package comprising a tray, a curve retention insert and a pouch are commonly employed. These methods are illustrated in Figures A and B. In both methods, it is normal and preferable to remove the catheter "luer fitting end" first from its package to minimise the risk of contamination by handling of those areas of the catheter (i.e. the shaft and curve) which are inserted into the body).

According to the first method (Fig. A), the sealed pouch is peeled back along its entire length to expose the catheter in the tray. The catheter is then removed from the tray by lifting the shaft from its retention tabs and at the same time removing the catheter curve from the curve retaining insert. If the curve retaining insert is held firmly in the tray, or moulded as part of the tray, the catheter must be lifted out of the insert. If, on the other hand, the insert is not attached to the tray, it may lift out with the curve as the catheter is removed from the tray. The curve portion may subsequently be removed from the insert. While this is the method of choice for removing a catheter from its package, it suffers from the disadvantage that it frequently requires more than one person to remove the catheter in order to avoid contamination, thus the removal process is somewhat cumbersome and time consuming.

The second method (Fig. B) is more often used in the busy hospital environment, where ease of handling and speed are of great importance. According to the second method, the seal on the pouch is broken by peeling back the pouch 10 cms or more at the luer fitting end. The tray is then withdrawn slightly from the pouch to give access to the luer fitting of the catheter. Then the luer fitting is grasped and the catheter is pulled quickly from the tray and pouch. That is, the catheter is pulled backwards through the package in a whiplike motion. While this releases the catheter quickly, it can result in considerable damage to both the shaft and the curve. Where a curve retainer insert is used, this method of removal is particularly difficult. If the retainer is held in the tray, the curve is subject to distortion when being pulled from the retainer, as it is held in position by tabs. As the choice of precise catheter curve shape and size required by the surgeon is usually critical to the particular operation being performed, any damage to the curve is likely to render a catheter useless. Also, the movement of the catheter along the tray may subject it to damage as the usual design of the tray is intended to facilitate removal of the shaft by lifting it out of its track, rather than the lateral movement along the track affected by this method.

U.S. Patent Specification No. 4,779,727 discloses a packaging system for a catheter having a tray formed with several tracks for receiving a catheter shaft and a interchangeable catheter tip-retaining insert which is held in a recess in the tray. The insert also carries several tracks, at least one of these tracks being matched so as to be continuous with a track in the tray portion, whereby the package can be used for retaining more than one catheter and/or catheter tips of varying shapes. This package must be opened by one of the methods described above, and a catheter retained within it is susceptible to damage as outlined.

The object of the present invention is to seek to provide a catheter package which protects the shaft and curve of a catheter from damage during storage and transit and from which the catheter may be easily removed without distortion or damage occurring to its shaft or curve.

According to the present invention, there is provided a package for a catheter, the catheter including an elongate catheter shaft with catheter elements at each end thereof, typically a luer fitting at one end and a curve at the other end, the package comprising a tray for accommodating the catheter shaft and an insert for retaining the curve, characterised in that the insert is movable relative to the tray along its longitudinal axis whereby on a longitudinal force being applied to the catheter, the insert and retained catheter curve move along the tray so as to allow the catheter to be removed from the package.

Advantageously, the insert and tray are provided with co-operating formations which enable the insert to be retained securely in the tray during storage and to be slidable within the tray on application of a longitudinal force.

Conveniently, the formations include a recess in the tray for accommodating the insert, the recess having a ramp leading from the surface of the recess to the surface of the tray to allow the insert to move freely from the recess on withdrawal of the catheter from the package.

Preferably, the upper edge of the recess is provided with at least one projection for preventing displacement of the insert from the recess except along the longitudinal axis of the tray.

Advantageously, the insert includes a flat surface having a skirt depending therefrom, the skirt having a rounded lower rim for engaging the ramp on withdrawal of the catheter from the package.

Conveniently, the junction between the surface and the skirt of the insert is rounded to facilitate movement of the insert along the package.

Preferably, the insert is provided with a track for receiving a catheter curve, the track having means for retaining the catheter curve in the track against said longitudinal force and any torsional force applied to the catheter shaft.

Advantageously, the retaining means comprise at least one tab for engaging the catheter.

Conveniently, the insert is adapted to retain a device for use with the catheter.

Preferably, the insert is provided with a main catheter track and at least two catheter tip retaining tracks, whereby the insert is usable to accommodate one of a number of catheter curve sizes.

Alternatively, the package includes a luer fitting insert for accommodating at least one catheter luer fitting, the luer fitting insert being slidable along the longitudinal axis of the tray.

Advantageously, the package includes a sealable pouch for enclosing the package.

The invention will now be described more particularly with reference to the accompanying drawings, which show, by way of example only, one embodiment of a catheter packaging system according to the invention.

In the drawings:
Figure 1 is a plan view of the tray;
Figure 2 is a sectional view of the tray along the line B-B of Figure 1;
Figure 3 is a sectional view of the tray along the line C-C of Figure 1;
Figure 4 is a sectional view of the tray along the line D-D of Figure 1;
Figure 5 is a plan view of a curve retention insert;
Figure 6 is a side elevation of the curve retention insert of Figure 5;
Figure 7 is an end elevation of the curve retention insert of Figure 5;
Figure 8 is a sectional view of the curve retention insert along the line A-A of Figure 5;
Figures 9a to 9d are each plan views of alternative embodiments of curve retention inserts; and
Figure 10a to 10d are sectional views of the curve retention inserts along, respectively, line E-E of Figure 9a, line F-F of Figure 9b, line G-G of Figure 9c and line H-H of Figure 9c.

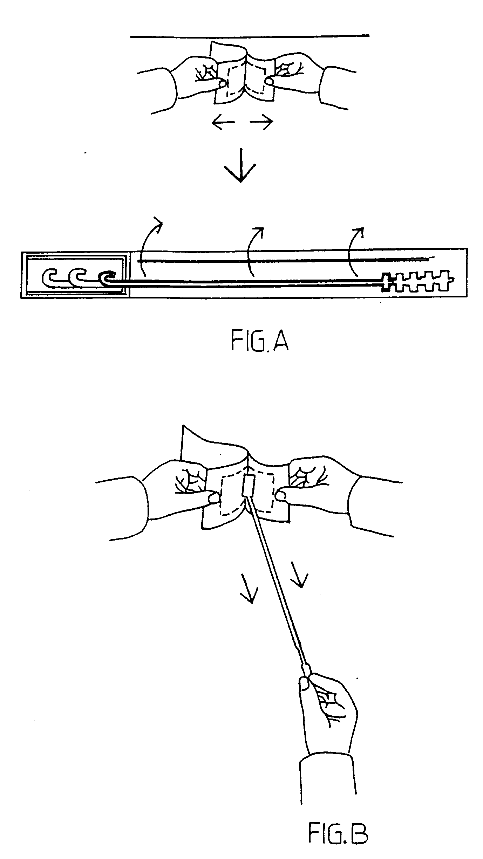

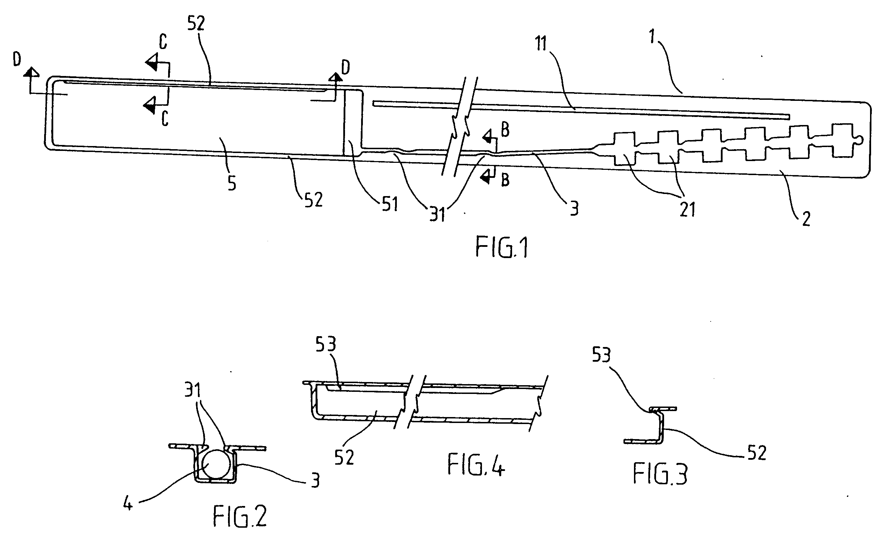

Referring now to Figures 1 to 4, the tray will now be described. The tray 1 is formed with a section 2 for accommodating a luer fitting in one of a plurality of luer lock locations 21, a track 3 for accommodating the shaft of a catheter 4 and a recess 5 for accommodating an insert. The junction between the upper surface of the tray 1 and the recess 5 is defined by a ramp 51. A reinforcing rib 11 is provided moulded into the underside of the tray 1 to provide resilience in the section of the tray 1 which accommodates the shaft of the catheter 4.

The upper-sides of the walls 52 of the recess 5 are provided with a lip 53, which serves to retain the insert in position. The track 3 is provided with three spaced apart tabs 31 which serve to retain the shaft of the catheter 4 in position within the track 3, after the catheter shaft has been pressed into the track 3. The size and configuration of the tabs 31 are chosen so as to provide easy loading of a catheter shaft 4 into the track 3, together with each removal of the shaft from the track when required. The tabs 31 provide a snap-fit retention for the shaft and are provided as staggered opposed pairs.

The appropriate luer lock location 21 is chosen for the particular catheter being packaged and depends on the length of the catheter.

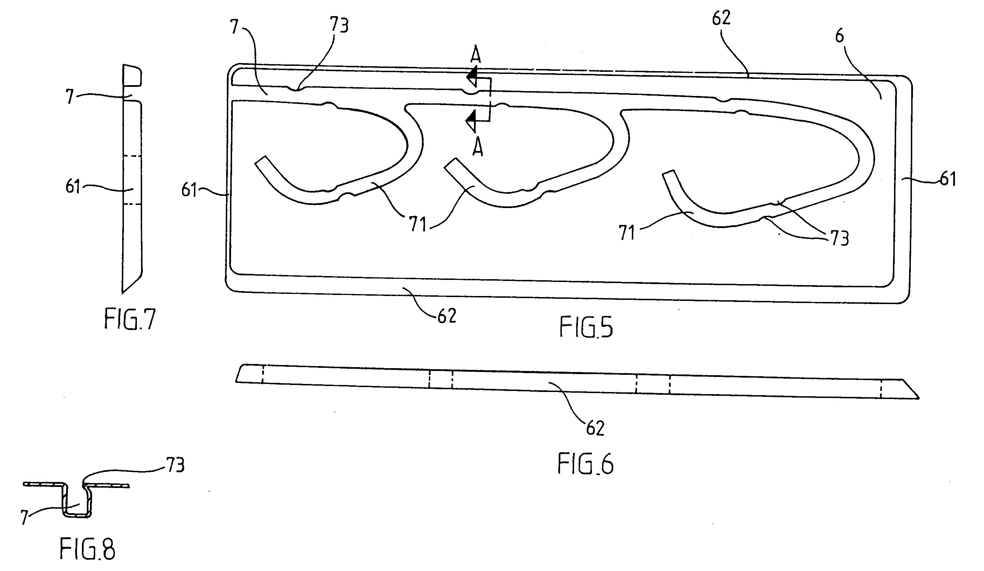

The curve retainer insert will now be described, with reference to Figures 5 to 8, which show the insert 6, having a main catheter track 7 leading to alternative catheter curve tip endings 71. The main track 7 and the endings 71 are provided with overhang tabs 73, provided as staggered opposed pairs, which act as retainers for the catheter shaft and ending. In addition to retaining the curve of a catheter in the track 7 and ending 71, the tabs 73 serve to prevent damage occurring to the curve during removal of the catheter from its package. Torque applied to the shaft at the end proximal the luer fitting tends to twist the curve and insert within the pouch, possibly causing the curve to lift out of the ending 71 with consequent curve distortion. The tabs 73 act to retain the curve in the ending 71 against the action of any torsional or other displacement force. Each of the end walls 61 and side walls 62 are outwardly sloped from top to bottom, as shown in the drawings, and all the corners are rounded.

The co-operation of the tray and insert and its use will now be described. To package a catheter, the curve retaining insert 6 is placed in the recess 5 of the tray 1. The catheter curve and shaft are then snap-fitted into their positions 71 and 3 respectively (the arrangement is such that the main catheter track 7 of the insert 6 lines up precisely with the track 3 of the tray 1). The luer fitting is then fitted into its appropriate luer lock location 21. The catheter in this package is then placed in a pouch, which is sealed, and the entire package is then sterilised by radiation, autoclaving or other means, as appropriate.

To remove the catheter from the package, the pouch is pealed back a few centimetres to expose the luer lock, which is then grasped and pulled laterally out of the package. On this traction movement, the shaft of the catheter 4 begins to slide through the track 3, bringing with it the curve retainer insert 6, which slides out from under the lip 53 of the recess 5, rides up the ramp 51 and from there slides over the surface of the tray 1 to the opening of the pouch. As the sloped leading end wall 61 rides up the ramp 51, the catheter shaft 4 is raised gently within the track 3 until it snaps out from beneath the tabs 31 and is released from the track. At this point, the catheter shaft and the insert can be readily removed from the tray, and subsequently the catheter curve can be gently removed from the insert 6. The sloped nature of the wall 61 assists the insert 6 in riding up the ramp 51, and the sloped and rounded nature of the walls 61, 62 in general of the insert 6 ensure that the insert 6 may move easily through the package without sticking, jamming or snagging on the tray 1 or the pouch.

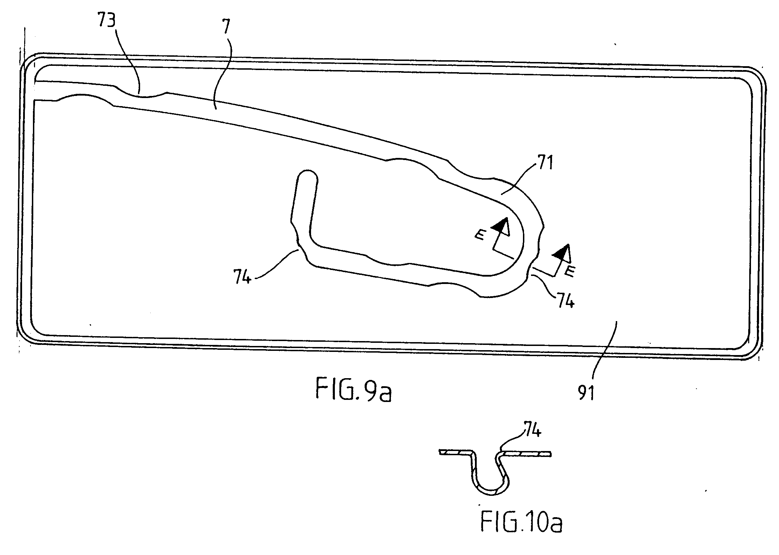

Figures 9a to 9d show four embodiments of curve retaining trays according to the invention, each of these trays being adapted to retain a single curve. The tray 91 of Figure 9a is suitable for retaining a curve of the type known as a Judkins left coronary. The track 7 of insert 91 is provided with two types of tabs, namely the overhang tabs 73 and ledge tab 74. Tab 74, which is shown in cross-section in Figure 10a, retains the bend of the curve in the track ending 71 against any dislodging force, such as torque applied to the main catheter shaft 4.

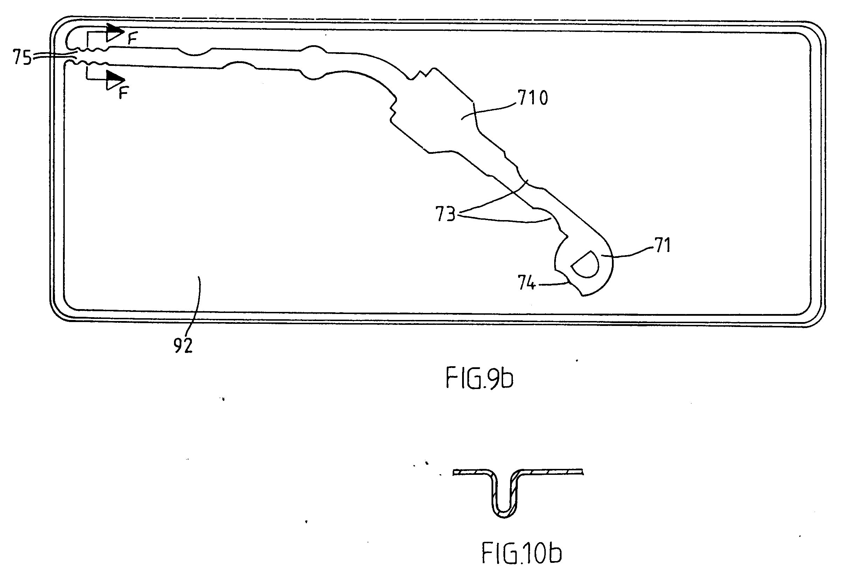

The insert 92 of Figure 9b is of a type for retaining a pigtail catheter. In addition to overhang tabs 73 and a ledge tab 74, this tray is further provided with grip or serrated tabs 75 which provide a retaining grip on a catheter shaft. This form of tab, shown in cross-section in Figure 10b, is particularly suitable for retaining catheters whose tips are relatively straight and which otherwise might tend to be pulled out of the insert on the application of the force applied by the operator to slide the catheter from the package. Insert 92 is also provided with an opened out portion 710 in the track 7, for accommodating a pigtail straightener. For insertion into a blood vessel, the tip of a pigtail catheter must be opened out. This is done using a pigtail straightener, which may be supplied with the package. The portion 710 is designed to receive such an instrument and to present it to the medical personnel in a convenient position adjacent to the pigtail curve itself.

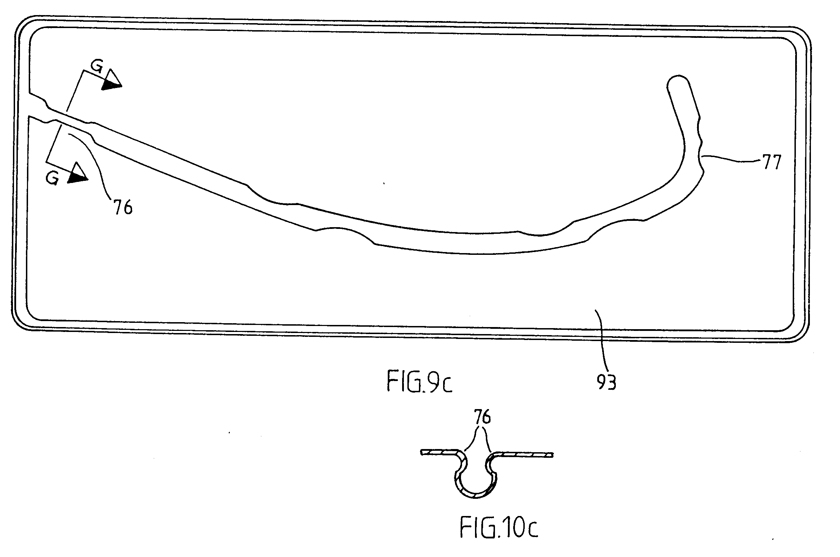

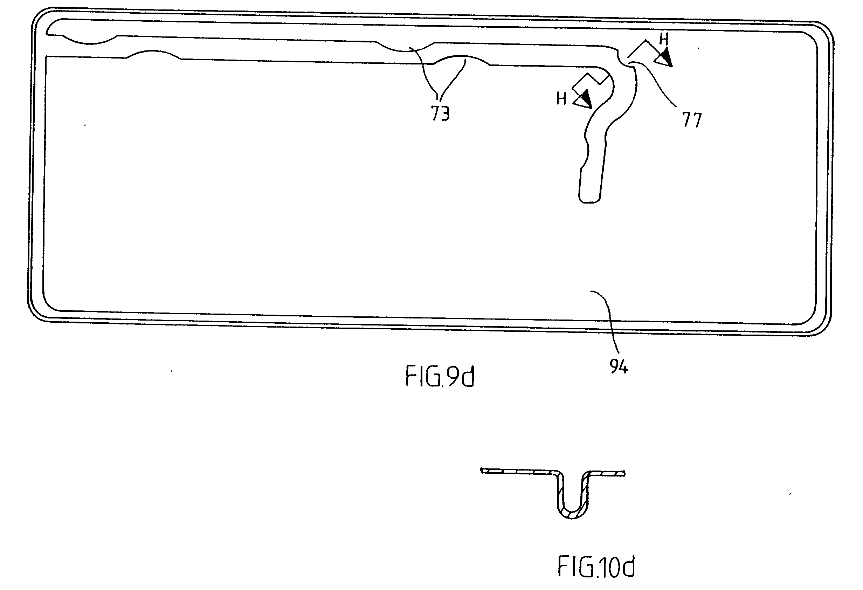

Figures 9c and 9d show two further embodiments of inserts 93 and 94. Insert 93 is adapted to receive a relatively straight curve and is provided with a variant form of gripping tab, the anchor tab 76, comprising two opposed gripping surfaces, as shown in cross-section in Figure 10c. The tip of the curve is supported by a pillar tab 77, which is shown in cross-section in Figure 10d. The insert 94 is designed to retain a curve whose shape is such that it is sufficiently retained by overhang tabs 73.

The overhang tab 73, ledge tab 74 and pillar tab 77 serve the function of preventing the catheter curve from lifting horizontally out of the curve track. The design of these tabs is such that a number of different catheter shaft sizes can be accommodated by a single size tab, for example French shaft sizes 5 to 8 could be retained by a given tab size. In the case of the serrated tab 75 or anchor tab 76, the tab acts to grip the shaft to resist longitudinal displacement of the curve within its track, therefore the tab size must be relatively closely matched to the shaft size and generally any given tab size would be suitable for use with only one or two different shaft sizes.

As exemplified by the insert 92 of Figure 9b, the inserts may be adapted to retain a further instrument which would be required for use in conjunction with a catheter. Such an additional instrument need not of course be retained within the catheter track itself, but may be provided on an appropriate recess formed elsewhere in the insert.

The recess in the main tray body for receiving the curve retaining insert may advantageously be longer than the length of the insert itself, to provide tolerance for manufacturing and other variations in catheter shaft length.

The tray and insert may be made from any suitable resilient materials, such as polystyrene or polyvinylchloride, or any combinations of suitable materials.

Finally, a second slidable insert may be provided for retaining the luer fitting. Such an insert may be formed with a number of luer lock locations for providing flexibility in packaging catheters of different lengths. A further recess is provided in the tray for receiving such a second insert.

## Claims

1. A package for a catheter (4), the catheter including an elongate catheter shaft with catheter elements at each end thereof, typically a luer fitting at one end and a curve at the other end, the package comprising a tray (1) for accommodating the catheter shaft and an insert for retaining the curve, characterised in that the insert (6) is movable relative to the tray along its longitudinal axis whereby on a longitudinal force being applied to the catheter, the insert and retained catheter curve move along the tray so as to allow the catheter to be removed from the package.

2. A package according to Claim 1, characterised in that the insert and tray are provided with co-operating formations which enable the insert to be retained securely in the tray during storage and to be slidable within the tray on application of a longitudinal force.

3. A package according to Claim 1 or Claim 2, characterised in that the formations include a recess in the tray for accommodating the insert, the recess having a ramp leading from the surface of the recess to the surface of the tray to allow the insert to move freely from the recess on withdrawal of the catheter from the package.

4. A package according to Claim 3, characterised in that the upper edge of the recess is provided with at least one projection for preventing displacement of the insert from the recess except along the longitudinal axis of the tray.

5. A package according to Claim 4, characterised in that the insert includes a flat surface having a skirt depending therefrom, the skirt having a rounded lower rim for engaging the ramp on withdrawal of the catheter from the package.

6. A package according to Claim 5, characterised in that the junction between the surface and the skirt of the insert is rounded to facilitate movement of the insert along the package.

7. A catheter according to Claim 6, characterised in that the insert is provided with a track for receiving a catheter curve, the track having means for retaining the catheter curve in the track against said longitudinal force and any torsional force applied to the catheter shaft.

8. A package according to Claim 7, characterised in that the retaining means comprise at least one tab for engaging the catheter.

9. A package according to any preceding claim, characterised in that the insert is adapted to retain a device for use with the catheter.

10. A package according to any preceding claim, characterised in that the insert is provided with a main catheter track and at least two catheter tip retaining tracks, whereby the insert is usable to accommodate one of a number of catheter curve sizes.

11. A package according to any preceding claim, characterised in that it includes a luer fitting insert for accommodating at least one catheter luer fitting, the luer fitting insert being slidable along the longitudinal axis of the tray.

12. A package according to any preceding claim, including a sealable pouch for enclosing the package.

## Patentansprüche

1. Verpackung für einen Katheter (4), der einen langgestreckten Katheterschaft enthält, an dessen beiden Enden Katheterelemente vorgesehen sind, typischerweise ein Lueranschluß am einen Ende und eine Biegung am anderen Ende, wobei die Verpackung eine flache Schale (1) zur Aufnahme des Katheterschaftes und einen Einsatz zum Halten der Biegung aufweist, dadurch gekennzeichnet, daß der Einsatz (6) gegenüber der Schale längs ihrer Längsachse bewegbar ist, so daß beim Ausüben einer Längskraft auf den Katheter sich der Einsatz und die gehaltene Katheterbiegung entlang der Schale bewegen, um so das Herausnehmen des Katheters aus der Verpackung zu erlauben.

2. Verpackung nach Anspruch 1, dadurch gekennzeichnet, daß der Einsatz und die Schale mit zusammenwirkenden Formungen versehen sind, die es ermöglichen, den Einsatz während der Aufbewahrung sicher in der Schale zu halten und ihn unter dem Einfluß einer angelegten Längskraft innerhalb der Schale gleiten zu lassen.

3. Verpackung nach Anspruch 1 oder Anspruch 2, dadurch gekennzeichnet, daß die Formungen eine Vertiefung in der Schale zur Aufnahme des Einsatzes enthalten, wobei diese Vertiefung eine Rampe hat, die von der Oberfläche der Vertiefung zur Oberfläche der Schale führt, um zu erlauben, daß sich der Einsatz beim Herausziehen des Katheters aus der Verpackung frei aus der Vertiefung bewegt.

4. Verpackung nach Anspruch 3, dadurch gekennzeichnet, daß das obere Ende der Vertiefung mit mindestens einem Vorsprung versehen ist, um eine Bewegung des Einsatzes aus der Vertiefung zu verhindern, ausgenommen eine Bewegung längs der Längsachse der Schale.

5. Verpackung nach Anspruch 4, dadurch gekennzeichnet, daß der Einsatz eine ebene Oberfläche mit einem von dieser nach unten stehenden Wand hat, die eine gerundete untere Kante aufweist, um beim Herausziehen des Katheters aus der Verpackung an der Rampe anzugreifen.

6. Verpackung nach Anspruch 5, dadurch gekennzeichnet, daß der Übergang zwischen der Oberfläche und dem Rand des Einsatzes gerundet ist, um die Bewegung des Einsatzes entlang der Verpackung zu erleichtern.

7. Katheter nach Anspruch 6, dadurch gekennzeichnet, daß der Einsatz mit einer Ablagebahn versehen ist, um eine Katheterbiegung aufzunehmen, wobei die Ablagebahn eine Vorrichtung aufweist, um die Katheterbiegung gegen die besagte Längskraft und gegen irgendeine auf den Katheterschaft ausgeübte Torsionskraft in der Ablagebahn zurückzuhalten.

8. Verpackung nach Anspruch 7, dadurch gekennzeichnet, daß die zurückhaltende Vorrichtung mindestens eine Nase zum Angreifen am Katheter aufweist.

9. Verpackung nach irgendeinem vorhergehenden Anspruch, dadurch gekennzeichnet, daß der Einsatz zum Halten eines mit dem Katheter zu verwendenden Gerätes ausgelegt ist.

10. Verpackung nach irgendeinem vorhergehenden Anspruch, dadurch gekennzeichnet, daß der Einsatz mit einer Hauptablagebahn für den Katheter und mindestens zwei Ablagebahnen zum Halten von Katheterspitzen versehen ist, so daß der Einsatz verwendbar ist, um Katheter mit unterschiedlichen Biegungsgröβen aufzunehmen.

11. Verpackung nach einem vorhergehenden Anspruch, dadurch gekennzeichnet, daß sie einen Lueranschluß-Einsatz enthält, um mindestens einen Katheter-Lueranschluß aufzunehmen, wobei der Lueranschluß-Einsatz entlang der Längsachse der Schale gleitbar ist.

12. Verpackung nach einem vorhergehenden Anspruch mit einer versiegelbaren Tasche zum Umschließen der Verpackung.

## Revendications

1. Emballage pour cathéter (4), le cathéter comprenant une tige de cathéter allongée ayant des éléments de cathéter à chaque extrémité, habituellement un embout "Luer" placé à une première extrémité et une partie courbe placée à l'autre extrémité, l'emballage comprenant un plateau (1) destiné à loger la tige du cathéter et un élément rapporté destiné à retenir la partie courbe, caractérisé en ce que l'élément rapporté (6) est mobile par rapport au plateau suivant son axe longitudinal, si bien que, lorsqu'une force longitudinale est appliquée au cathéter, l'élément rapporté et la partie courbe de cathéter qui est retenue se déplacent le long du plateau en permettant une extraction du cathéter de l'emballage.

2. Emballage selon la revendication 1, caractérisé en ce que l'élément rapporté et le plateau ont des organes conformés coopérants qui permettent la retenue immobile de l'élément rapporté dans le plateau pendant le stockage et son coulissement dans le plateau lors de l'application d'une force longitudinale.

3. Emballage selon la revendication 1 ou 2, caractérisé en ce que les organes conformés comprennent une cavité formée dans le plateau pour le logement de l'élément rapporté, la cavité ayant une rampe partant de la surface de la cavité jusqu'à la surface du plateau de manière qu'elle permette un déplacement libre de l'élément rapporté de la cavité lors de l'extraction du cathéter de l'emballage.

4. Emballage selon la revendication 3, caractérisé en ce que le bord supérieur de la cavité a au moins une saillie destinée à empêcher le déplacement de l'élément rapporté en dehors de la cavité sauf suivant l'axe longitudinal du plateau.

5. Emballage selon la revendication 4, caractérisé en ce que l'élément rapporté comporte une surface plate ayant une jupe dépassant ce celle-ci, la jupe ayant un rebord inférieur arrondi destiné à coopérer avec la rampe lors de l'extraction du cathéter de l'emballage.

6. Emballage selon la revendication 5, caractérisé en ce que la jonction de la surface et de la jupe de l'élément rapporté est arrondie afin qu'elle facilite le déplacement de l'élément rapporté le long de l'emballage.

7. Cathéter selon la revendication 6, caractérisé en ce que l'élément rapporté comporte un logement allongé destiné à loger une partie courbe de cathéter, le logement allongé ayant un dispositif de retenue de la partie courbe du cathéter dans le logement soumis à ladite force longitudinale et à une force quelconque de torsion appliquée à la tige du cathéter.

8. Emballage selon la revendication 7, caractérisé en ce que le dispositif de retenue comprend au moins une patte destinée à coopérer avec le cathéter.

9. Emballage selon l'une quelconque des revendications précédentes, caractérisé en ce que l'élément rapporté est adapté à retenir un dispositif destiné à être utilisé avec le cathéter.

10. Emballage selon l'une quelconque des revendications précédentes, caractérisé en ce que l'élément rapporté a une voie principale et au moins deux voies de retenue de bout de cathéter, si bien que l'élément rapporté peut être utilisé pour le logement d'une partie courbe de cathéter ayant une dimension choisie parmi un certain nombre de dimensions différentes.

11. Emballage selon l'une quelconque des revendications précédentes, caractérisé en ce qu'il comprend un élément rapporté pour embout "Luer" destiné à loger au moins un embout "Luer" de cathéter, l'élément rapporté d'embout "Luer" pouvant coulisser le long de l'axe longitudinal du plateau.

12. Emballage selon l'une quelconque des revendications précédentes, comprenant une poche qui peut être scellée et qui est destinée à entourer l'emballage.
